(19) **Europäisches Patentamt / European Patent Office / Office européen des brevets**

(11) **EP 4 618 002 A1**

(12) # EUROPEAN PATENT APPLICATION
published in accordance with Art. 153(4) EPC

(43) Date of publication:
**17.09.2025 Bulletin 2025/38**

(21) Application number: **23888449.8**

(22) Date of filing: **18.10.2023**

(51) International Patent Classification (IPC):
*G06T 7/00* (2017.01)     *G06V 10/82* (2022.01)
*G01N 23/046* (2018.01)     *G01N 23/083* (2018.01)

(52) Cooperative Patent Classification (CPC):
**G01N 23/046; G01N 23/083; G06T 7/00;
G06V 10/82**

(86) International application number:
**PCT/JP2023/037699**

(87) International publication number:
**WO 2024/101098 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.11.2022 JP 2022179731**

(71) Applicants:
• **IHI Corporation
Tokyo 135-8710 (JP)**
• **Katayanagi Institute
Hachioji-shi, Tokyo 192-0982 (JP)**

(72) Inventors:
• **KAMAGATA, Takanori
Tokyo 135-8710 (JP)**
• **SHICHIJO, Naohiro
Hachioji-shi, Tokyo 192-0982 (JP)**
• **SHIBATA, Chihiro
Hachioji-shi, Tokyo 192-0982 (JP)**

(74) Representative: **MERH-IP Matias Erny Reichl
Hoffmann
Patentanwälte PartG mbB
Paul-Heyse-Straße 29
80336 München (DE)**

(54) **IDENTIFICATION DEVICE FOR IDENTIFYING STRUCTURAL ELEMENT INCLUDED IN COMPOSITE MATERIAL, IDENTIFICATION METHOD, AND IDENTIFICATION PROGRAM**

(57)     An identification device, an identification method, and an identification program receive a physical quantity distribution inside a member that includes multiple structural elements, generate a plurality of likelihood data indicating likelihood of the structural element to which a voxel belongs based on a plurality of two-dimensional images indicating the physical quantity distribution in a plurality of different cross sections intersecting the voxel, calculate a weighted sum of the likelihood based on the plurality of likelihood data, and set a label for identifying the structural element for which the likelihood is maximized in the voxel based on the weighted sum.

# FIG. 1

**10**
IMAGING UNIT

**20**

**21**
RECEIVING UNIT

DATABASE
**23**

CONTROLLER
**25**

**251** CROSS SECTION DESIGNATING UNIT

**253** TWO-DIMENSIONAL IMAGE ACQUIRING UNIT

**255** LIKELIHOOD DATA GENERATING UNIT

**257** CALCULATING UNIT

**259** LABEL SETTING UNIT

OPERATING UNIT
**27**

PRESENTING UNIT
**29**

**Description**

TECHNICAL FIELD

The present disclosure relates to an identification device, an identification method, and an identification program for identifying structural elements included in a composite material.

BACKGROUND ART

Patent Literature 1 discloses a technique for analyzing the orientation of fiber bundles based on a three-dimensional image obtained by imaging a fiber-reinforced composite material with an X-ray Computed Tomography device. According to this technique, the direction in which X yarn and Y yarn perpendicularly cross each other is determined as a reference direction from a binarized three-dimensional image of a fabric made of fiber bundles of X yarn, Y yarn, and Z yarn. Then, a directional distance method is applied on a reference plane perpendicular to the reference direction to estimate the orientation of the fiber bundles of the X yarn and the Y yarn.

CITATION LIST

PATENT LITERATURE

Patent Literature 1: International Publication No. 2016/052489

SUMMARY OF THE INVENTION

TECHNICAL PROBLEM

The technique disclosed in Patent Literature 1 has a problem that the accuracy of identifying a structural element contained in a composite material decreases when the composite material has a complex three-dimensional structure. Furthermore, if an attempt is made to improve the accuracy of identifying the structural element, a problem arises in that the cost and time required for the identification process increase.

The present disclosure has been made in view of the above-mentioned circumstances. In other words, the present disclosure aims to provide an identification device, an identification method, and an identification program that can improve the accuracy of identifying a structural element contained in the composite material while suppressing increases in cost and time required for identifying the structural element.

SOLUTION TO PROBLEM

An identification device of the present disclosure comprises a receiving unit configured to receive a physical quantity distribution inside a member that includes multiple structural elements, and a controller configured to identify the structural element to which a voxel included in the member belongs based on the physical quantity distribution. The controller generates a plurality of likelihood data indicating likelihood of the structural element to which the voxel belongs based on a plurality of two-dimensional images indicating the physical quantity distribution in a plurality of different cross sections intersecting the voxel, calculates a weighted sum of the likelihood based on the plurality of likelihood data generated for each voxel, and sets a label for identifying the structural element for which the likelihood is maximized in the voxel based on the weighted sum.

The controller may perform a semantic segmentation on the two-dimensional image to generate the likelihood data.

The semantic segmentation may be performed using a learning model obtained by performing machine learning based on teaching data.

The controller may generate the plurality of likelihood data based on the plurality of two-dimensional images indicating the physical quantity distribution in three or more cross sections orthogonal to each other.

The controller may perform threshold processing based on the physical quantity distribution to identify whether or not the voxel is a void.

The structural element may be at least one of one or more fiber bundles, a matrix, and a void.

The receiving unit may receive as the physical quantity distribution a distribution representing an absorbance of X-rays at each point inside the member, the distribution being acquired by an X-ray Computed Tomography device.

An identification method of the present disclosure identifies a structural element to which a voxel included in a member belongs, the member that includes multiple structural elements, receives a physical quantity distribution inside the member, generates a plurality of likelihood data indicating likelihood of the structural element to which the voxel belongs based on a plurality of two-dimensional images indicating the physical quantity distribution in a plurality of different cross sections intersecting the voxel, calculates a weighted sum of the likelihood based on the plurality of likelihood data generated for each voxel, and sets a label for identifying the structural element for which the likelihood is maximized in the voxel based on the weighted sum.

An identification program of the present disclosure identifies a structural element to which a voxel included in a member belongs, the member that includes multiple structural elements. Then, the computer is caused to execute a step of receiving a physical quantity distribution inside the member. The computer is caused to execute a step of generating a plurality of likelihood data indicating likelihood of the structural element to which the voxel belongs based on a plurality of two-dimensional images indicating the physical quantity distribution in a plurality of different cross sections intersecting the voxel. Then, the computer is caused to execute a step of calculating a weighted sum of the likelihood based on the plurality of likelihood data generated for each voxel, and a step of setting a label for identifying the structural element for which the likelihood is maximized in the voxel based on the weighted sum.

## ADVANTAGEOUS EFFECTS OF INVENTION

According to the present disclosure, it is possible to improve the accuracy of identifying a structural element contained in the composite material while suppressing increases in cost and time required for identifying the structural element.

## BRIEF DESCRIPTION OF DRAWINGS

[Fig. 1] Fig. 1 is a block diagram showing a configuration of an identification device according to an embodiment of the present disclosure.
[Fig. 2] Fig. 2 is a flowchart showing a processing procedure of the identification device according to the embodiment of the present disclosure.

## DESCRIPTION OF EMBODIMENTS

Hereinafter, some exemplary embodiments will be described with reference to the drawings. In addition, common parts in the figures are given the same reference numerals, and duplicated explanations will be omitted.

[Subject of processing by the identification device]

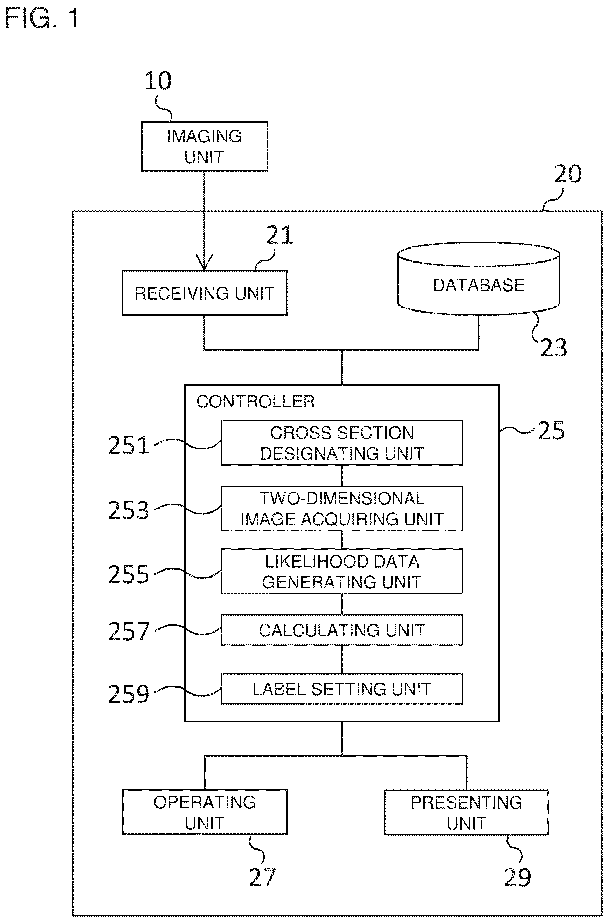

As shown in Fig. 1, the identification device 20 is connected to the imaging unit 10. Here, the imaging unit 10 irradiates the member to be measured with electromagnetic waves (e.g., X-rays) and detects the electromagnetic waves that are transmitted through the member to obtain the physical quantity distribution inside the member.

For example, the imaging unit 10 is an X-ray Computed Tomography device. In this case, the physical quantity distribution inside the member is acquired by imaging with the imaging unit 10. The "physical quantity distribution" inside the member represents the luminance at each point inside the member. The luminance at each point inside the member is associated with a "physical quantity" at each point, such as the absorbance of X-rays at each point or the degree of attenuation of X-rays. The luminance at each point inside the member itself may be treated as a "physical quantity."

The imaging unit 10 may acquire the luminance at each point on a predetermined cross section of the member, and generate a two-dimensional image corresponding to the cross section. Here, the predetermined cross section may be arbitrarily set. For example, the coordinate axes of the stationary system of the member may be the X-axis, the Y-axis, and the Z-axis, and the predetermined cross section may be set as a plane parallel to any of the XY plane, the YZ plane, and the XZ plane. Furthermore, the imaging unit 10 may set a plurality of cross sections parallel to each other, group two-dimensional images generated for each cross section, and generate the physical quantity distribution in a three-dimensional space.

The member (composite material) from which the physical quantity distribution is acquired by the imaging unit 10 includes a plurality of structural elements. For example, the structural elements are at least one of one or a plurality of fiber bundles, a matrix, and a void. The member may be obtained by impregnating a molded reinforcing material with a matrix (base material) to compound the reinforcing material and the matrix. The reinforcing material is, for example, graphite, boron nitride, or ceramics such as silicon carbide. The reinforcing material may be a resin such as Kevlar (registered trademark), or an appropriate metal or alloy. The reinforcing material may be made of a plurality of fiber bundles.

The matrix is, for example, a thermosetting resin, a thermoplastic resin, or a ceramic such as silicon carbide. A member whose matrix is made of ceramic is particularly called a ceramic matrix composite (CMC). A void is a gap-like defect that occurs in a member.

The identification device 20 acquires a physical quantity distribution inside a member including a plurality of structural elements, using the imaging unit 10. As will be described later, the identification device 20 has a function of identifying the structural element to which a voxel included in the member belongs, based on the physical quantity distribution acquired by the imaging unit 10.

A voxel is the smallest unit of data for expressing a solid object on a computer. For example, the voxel may be a cube, which is a unit element of an orthogonal lattice. The voxel may also be another type of polyhedron. On a computer, the member including multiple structural elements is expressed as a set of voxels, and each voxel has a scalar value and a vector value. By assigning the above-mentioned physical quantity to each voxel, the entire set of voxels can express the above-mentioned "physical quantity distribution."

[Configuration of the identification device]

Fig. 1 is a block diagram showing the configuration of the identification device 20. The identification device 20 comprises a receiving unit 21, a database 23 (storage unit), a controller 25, an operating unit 27, and a presenting unit 29. The controller 25 is connected to the receiving unit 21, the database 23, the operating unit 27, and the presenting unit 29 so as to be able to communicate with them.

In addition, the operating unit 27 and the presenting unit 29 may be provided in the identification device 20 itself, or may be installed outside the identification device 20 and connected to the identification device 20.

The receiving unit 21 is connected wirelessly or wired to be able to communicate with the imaging unit 10. The receiving unit 21 receives data indicating a physical quantity distribution inside the member from the imaging unit 10. The receiving unit 21 may receive two-dimensional images generated for each predetermined cross section of the member, or may receive data indicating a physical quantity distribution in a three-dimensional space obtained by grouping two-dimensional images.

Furthermore, the receiving unit 21 may receive data indicating the physical quantity distribution from a storage medium or the like. In this case, the data indicating the physical quantity distribution stored in the storage medium or the like may be measured using the imaging unit 10 that is not connected to the identification device 20.

Alternatively, the receiving unit 21 may receive design data of the member or three-dimensional measurement data of the member. The design data of the member is, for example, data generated by CAD (Computer-aided design) when designing the member. The three-dimensional measurement data of the member is, for example, data indicating the outer shape of the member obtained by actually measuring the member using a three-dimensional measuring device.

The database 23 stores data indicating the physical quantity distribution received by the receiving unit 21. That is, the database 23 may store two-dimensional images generated for each predetermined cross section of the member, or may store data indicating the physical quantity distribution in three-dimensional space. In addition, the database 23 may store a timestamp specifying the date and time of imaging by the imaging unit 10, an identification number specifying the member that was the subject of measurement by the imaging unit 10, and the like, together with the received data.

The operating unit 27 is an input device that can be operated by a user, such as a monitor or maintenance person of the evaluation system. For example, the operating unit 27 is a keyboard, a mouse, a trackball, a touch panel, or the like. The operating unit 27 is not limited to the examples given here. The content of the user's operation input via the operating unit 27 is transmitted to the controller 25.

The presenting unit 29 displays the information generated by the controller 25. The presenting unit 29 may also present to the user a label that identifies a structural element to which the voxel contained in the member belongs, the label being set by the controller 25 (described later). Alternatively, the presenting unit 29 may present to the user a label that identifies whether or not the voxel is a void. For example, the presenting unit 29 may be a display that displays figures and characters by combining a plurality of display pixels. The presenting unit 29 is not limited to the examples given here.

The identification device 20 may include an output unit that outputs information generated by the controller 25 to the outside of the identification device 20. The output unit may output the label that identifies the structural element to which the voxel belongs. The output unit may also output the label that identifies whether or not the voxel is a void.

The controller 25 (control unit) is a general-purpose computer equipped with a CPU (Central Processing Unit), a memory, and an input/output unit. A computer program (identification program) for functioning as the identification device 20 is installed in the controller 25. By executing the computer program, the controller 25 functions as a plurality of information processing circuits (251, 253, 255, 257, 259) equipped in the identification device 20. The computer program may be stored in a storage medium that can be read and written by a computer. Alternatively, the computer program may be capable of being distributed via a telecommunications line.

In the present disclosure, an example is shown in which multiple information processing circuits (251, 253, 255, 257, 259) are realized by software. However, it is also possible to prepare dedicated hardware for executing each information process shown below to configure the information processing circuits (251, 253, 255, 257, 259). In addition, the multiple information processing circuits (251, 253, 255, 257, 259) may be configured by individual hardware. Furthermore, the information processing circuits (251, 253, 255, 257, 259) may also be used as a control unit used for monitoring or controlling the imaging unit 10.

As shown in Fig. 1, the controller 25 includes a cross section designating unit 251, a two-dimensional image acquiring unit 253, a likelihood data generating unit 255, a calculating unit 257, and a label setting unit 259 as a plurality of information processing circuits (251, 253, 255, 257, 259).

The cross section designating unit 251 designates a cross section that intersects with a voxel for which likelihood data, which will be described later, is to be generated. The cross section designating unit 251 designates a plurality of different cross sections. For example, the cross section designating unit 251 may designate three or more cross sections that are mutually orthogonal. That is, the cross section designating unit 251 may designate the XY plane, the YZ plane, and the XZ plane with the position of the voxel as the origin. The direction of the cross section designated by the cross section designating unit 251 is consistent with the direction of the cross section when creating a learning model, which will be described later.

The two-dimensional image acquiring unit 253 acquires a two-dimensional image corresponding to the cross section specified by the cross section designating unit 251. When a two-dimensional image corresponding to the specified cross section is stored in the database 23, the two-dimensional image acquiring unit 253 may read out the two-dimensional image from the database 23. When data indicating a physical quantity distribution in a three-dimensional space is stored in the database 23, the two-dimensional image acquiring unit 253 may execute a multiplanar reconstruction method. The two-dimensional image acquiring unit 253 may acquire a two-dimensional image corresponding to the specified cross section by executing the multiplanar reconstruction method based on the data indicating a physical quantity distribution in a three-dimensional space.

The likelihood data generating unit 255 generates likelihood data indicating the likelihood of the structural element to which the voxel belongs, based on the two-dimensional image acquired by the two-dimensional image acquiring unit 253. The likelihood data generating unit 255 generates, for each voxel, likelihood data in the number equal to the number of cross sections specified by the cross section designating unit 251. Note that the likelihood data generating unit 255 may perform a process of generating likelihood data for a plurality of voxels.

Here, "likelihood data" refers to data indicating the probability that the voxel belongs to each of the structural elements included in the member. For example, when the structural elements are composed of fiber bundles, matrices, and voids, the "likelihood data" is expressed as "fiber bundle A%, matrix B%, void C%" (where A+B+C=100). When there is not one type of fiber bundle but multiple types (for example, when there are fiber bundles of X yarn, Y yarn, and Z yarn), the likelihood data may indicate the probability for each type of fiber bundle.

The likelihood data generating unit 255 may generate likelihood data by performing semantic segmentation on the two-dimensional image. In the semantic segmentation, the voxel (target voxel) for which likelihood data is generated and voxels (surrounding voxels) located around the target voxel are referenced. The likelihood data generating unit 255 generates likelihood data indicating the likelihood of the structural element to which the target voxel belongs based on physical quantities assigned to the target voxel and the surrounding voxels. The positional relationship between the target voxel and the surrounding voxels referenced when generating the likelihood data may be determined by the size, shape, etc. of the kernel used for convolution in the semantic segmentation.

The relationship between the physical quantity assigned to the target voxel and the surrounding voxels and the generated likelihood data may be defined by the learning model obtained by performing machine learning based on the teaching data. In other words, the semantic segmentation may be performed using a learning model obtained by performing machine learning based on the teaching data. Here, the "teaching data" refers to a set of voxels to which labels identifying structural elements are set by a person (expert) who understands the internal structure of the member including multiple structural elements.

When the learning model is a neural network, the neural network includes an input layer (or kernel), an output layer to which an output value is output, and at least one or more hidden layers provided between the input layer and the output layer, and signals are propagated in the order of the input layer, hidden layer, and output layer. Each of the input layer, hidden layer, and output layer is composed of one or more units. The units between the layers are connected to each other, and each unit has an activation function (e.g., a sigmoid function, a normalized linear function, a softmax function, etc.). A weighted sum is calculated based on multiple inputs to the unit, and the value of the activation function with the sum value as a variable becomes the output of the unit. For example, in machine learning, the weights used when calculating the sum in each unit of the neural network are adjusted as parameters related to the learning model.

When machine learning is performed in the likelihood data generating unit 255, the physical quantities assigned to the target voxel and the surrounding voxels in the teaching data are input to the input layer of the neural network constituting the learning model. The parameters related to the learning model are adjusted so that the error between the value output from the output layer when the physical quantities are input to the input layer and the label identifying the structural element set for the target voxel in the teaching data is reduced.

In order to minimize an error related to the output of the neural network, for example, the likelihood data generating unit 255 may use a gradient descent method, a stochastic gradient descent method, etc. The likelihood data generating unit 255 may use an error backpropagation method for gradient calculation in the gradient descent method or the stochastic gradient descent method.

In machine learning using neural networks, generalization performance (the ability to discriminate between unknown data) and overfitting (the phenomenon in which the learning model fits the data used to create it but the generalization performance does not improve) can become problems.

Therefore, in creating the learning model in the likelihood data generating unit 255, a method such as regularization that restricts the degree of freedom of weights during learning may be used to alleviate overfitting. In addition, a method such as dropout that probabilistically selects units in the neural network and invalidates other units may be used. Furthermore, in order to improve generalization performance, a method such as data regularization, data standardization, and data expansion that eliminate bias in data may be used.

In addition, the learning model is created independently for each direction of the cross section. For example, when the XY plane, the YZ plane, and the XZ plane are set as cross sections with the position of the voxel as the origin, three types of learning models are created: a learning model corresponding to the XY plane, a learning model corresponding to the YZ plane, and a learning model corresponding to the XZ plane.

In creating the learning model corresponding to the selected plane, a set of voxels with labels that identify structural elements are cut by a plane parallel to the selected plane. The physical quantities of the voxels that appear on the cut surface are then used as teaching data. In this way, different teaching data can be obtained by switching the selected plane, so that the learning model can be created independently for each direction of cross section.

The likelihood data generating unit 255 performs semantic segmentation using the learning model created as described above to generate likelihood data based on the two-dimensional image. Note that the learning model is selected so that the direction of the cross section for which the learning model was created matches the direction of the cross section corresponding to the two-dimensional image to be processed by the semantic segmentation.

The calculating unit 257 calculates a weighted sum of the likelihoods based on a plurality of pieces of likelihood data generated for each voxel included in the member.

For example, let us assume that three pieces of likelihood data are generated for each voxel based on a two-dimensional image for the XY plane, a two-dimensional image for the YZ plane, and a two-dimensional image for the XZ plane. Let the likelihood data for the XY plane be "fiber bundle A1%, matrix B1%, void C1%." Let the likelihood data for the YZ plane be "fiber bundle A2%, matrix B2%, void C2%." Let the likelihood data for the XZ plane be "fiber bundle A3%, matrix B3%, void C3%."

In this case, the calculating unit 257 calculates the weighted sum of the likelihoods as "fiber bundle AS%, matrix BS%, void CS%", where

$$AS = A1*w1 + A2*w2 + A3*w3,$$

$$BS = B1*w1 + B2*w2 + B3*w3,$$

$$CS = C1*w1 + C2*w2 + C3*w3.$$

Furthermore, w1, w2, and w3 are "weights" used when calculating the weighted sum. The "weights" may be set manually or automatically based on the shape factors of the members.

Alternatively, the calculating unit 257 may perform threshold processing based on the physical quantity distribution to identify whether or not a voxel is a void. For example, the calculating unit 257 may determine that the target voxel is a void when the physical quantity assigned to the target voxel is equal to or greater than a predetermined threshold. The calculating unit 257 may determine that the target voxel is not a void when the physical quantity assigned to the target voxel is less than the predetermined threshold.

The label setting unit 259 sets to the voxel a label that identifies the structural element to which the voxel belongs that has the maximum likelihood based on the weighted sum. For example, if the weighted sum of the likelihood is calculated as "fiber bundle AS%, matrix BS%, void CS%, " the label setting unit 259 sets to the voxel a label that identifies the structural element having the maximum value among AS, BS, and CS.

The label setting unit 259 may set a label for identifying a structural element to the voxel by combining the results of the threshold processing performed by the calculating unit 257. In particular, since a void is identified based on a plurality of different processes, namely, the process for identifying based on a two-dimensional image and the threshold processing, it is possible to improve the accuracy in identifying whether or not a voxel is a void.

[Processing procedure of identification device]

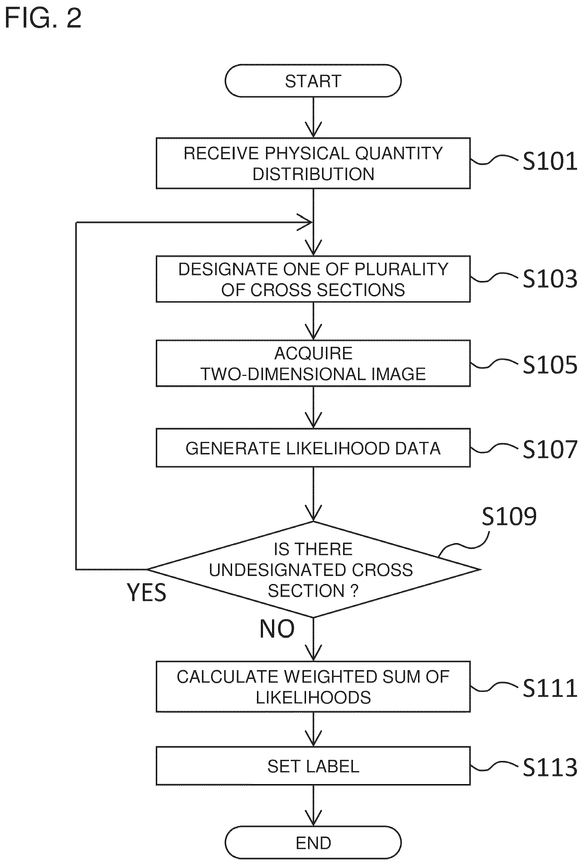

Next, a processing procedure of the identification device 20 according to the present disclosure will be described with reference to the flowchart of Fig. 2. The processing of the flowchart shown in Fig. 2 is started based on, for example, a user instruction.

In step S101, the receiving unit 21 receives the physical quantity distribution from the imaging unit 10.

In step S103, the cross section designating unit 251 designates a cross section intersecting with a voxel for which likelihood data is to be generated. To this end, the cross section designating unit 251 designates one of a plurality of cross sections (undesignated cross sections).

In step S105, the two-dimensional image acquiring unit 253 acquires a two-dimensional image corresponding to the cross section designated by the cross section designating unit 251.

In step S107, the likelihood data generating unit 255 generates likelihood data indicating the likelihood of the structural element to which the voxel belongs, based on the two-dimensional image acquired by the two-dimensional image acquiring unit 253.

In step S109, the cross section designating unit 251 determines whether or not there is an undesignated cross section among the multiple cross sections. If there is an undesignated cross section (YES in step S109), the process returns to step S103.

If there are no unspecified cross sections (NO in step S109), in step S111, the calculating unit 257 calculates a weighted sum of the likelihoods based on multiple pieces of likelihood data generated for each voxel included in the member.

In step S113, the label setting unit 259 sets, to the voxel, a label that identifies the structural element to which the voxel belongs that maximizes the likelihood of the structural element being the one to which the voxel belongs, based on the weighted sum. Then, the process of the flowchart shown in Fig. 2 ends.

[Effect of Embodiments]

As explained in detail above, an identification device, an identification method, and an identification program according to the present disclosure identify a structural element to which a voxel included in a member belongs, the member that includes multiple structural elements. Then, the identification device, the identification method, and the identification program receive a physical quantity distribution inside the member, generate a plurality of likelihood data indicating likelihood of the structural element to which the voxel belongs based on a plurality of two-dimensional images indicating the physical quantity distribution in a plurality of different cross sections intersecting the voxel, calculate a weighted sum of the likelihood based on the plurality of likelihood data generated for each voxel, and set a label for identifying the structural element for which the likelihood is maximized in the voxel based on the weighted sum.

This makes it possible to improve the accuracy of identifying structural elements contained in a composite material while suppressing increases in cost and time required for identifying the structural elements. In addition, since likelihood data is generated for each of a plurality of different cross sections to identify the structural element to which the voxel belongs, the accuracy of identification can be improved.

When identifying a structural element based on likelihood data only in one cross section, the continuity of the identification in the direction perpendicular to the cross section is lost, and the accuracy of the identification may deteriorate. On the other hand, according to the identification device, the identification method, and the identification program of the present disclosure, likelihood data is generated in a plurality of different cross sections to identify the structural element to which the voxel belongs, so that the continuity of the identification is maintained and the accuracy of the identification can be improved.

Furthermore, since the accuracy of identification can be improved, it is possible to generate three-dimensional data used for simulating the material structure of a composite member with high accuracy.

Further, the identification device, the identification method, and the identification program according to the present disclosure may perform a semantic segmentation on the two-dimensional image to generate the likelihood data. This allows labels that identify structural elements to be automatically set to voxels instead of a person (expert) who understands the internal structure of the member including multiple structural elements. As a result, the cost and time required to identify structural elements included in a composite material can be reduced.

Furthermore, in the identification device, the identification method, and the identification program according to the present disclosure, the semantic segmentation may be performed using a learning model obtained by performing machine learning based on teaching data. This allows the know-how of experts to be incorporated as the learning model, reducing the cost and time required to identify structural elements contained in a composite material.

Further, the identification device, the identification method, and the identification program according to the present disclosure may generate the plurality of likelihood data based on the plurality of two-dimensional images indicating the physical quantity distribution in three or more cross sections orthogonal to each other. This makes it possible to improve the accuracy in identifying the structural element to which a voxel belongs based on multiple pieces of likelihood data.

Furthermore, the identification device, the identification method, and the identification program according to the present disclosure may perform threshold processing based on the physical quantity distribution to identify whether or not the voxel is a void. This makes it possible to identify voids contained in the composite material and evaluate the void content ratio.

Further, in the identification device, the identification method, and the identification program according to the present disclosure, the structural element is at least one of one or more fiber bundles, a matrix, and a void. It is possible to identify structural elements for composite materials consisting of fiber bundles, matrix, and voids, and to generate three-dimensional data with high accuracy for use in material structure simulation of composite material.

Furthermore, in the identification device, the identification method, and the identification program according to the present disclosure, the receiving unit receives as the physical quantity distribution a distribution representing an absorbance of X-rays at each point inside the member, the distribution being acquired by an X-ray Computed Tomography device. This allows the physical quantity distribution to be acquired by penetrating the inside of the member. As a result, it is possible to identify a plurality of structural elements included in the composite member. Furthermore, it is possible to generate three-dimensional data to be used for a material structure simulation of the composite member using the identification result.

Respective functions described in the present disclosure may be implemented by one or plural processing circuits. The processing circuits include programmed processors, electrical circuits, etc., as well as devices such as application specific integrated circuits (ASIC) and circuit components arranged to perform the described functions, etc.

According to the present disclosure, it is possible to improve the accuracy of identification while suppressing increases in cost and time required for identifying the structural element contained in the composite material. As a result, it is possible to promote the production of high-quality composite materials. Therefore, it is possible to contribute to, for example, Goal 9 of the Sustainable Development Goals (SDGs) led by the United Nations, "Build resilient infrastructure, promote inclusive and sustainable industrialization and foster innovation."

Although some embodiments have been described, it is possible to modify or transform the embodiments based on the above disclosure contents. All components of the above embodiment, and all the features described in the claims, may be individually extracted and combined as long as they do not contradict each other.

This application claims priority based on Japanese Patent Application No. 2022-179731 filed on November 9, 2022, and the entire content of this application is incorporated herein by reference.

REFERENCE SIGNS LIST

| 10 | imaging unit |
| 20 | identification device |
| 21 | receiving unit |
| 23 | database |
| 25 | controller |
| 27 | operating unit |
| 29 | presenting unit |
| 251 | cross section designating unit |
| 253 | two-dimensional image acquiring unit |
| 255 | likelihood data generating unit |
| 257 | calculating unit |
| 259 | label setting unit |

**Claims**

1. An identification device comprising:

   a receiving unit configured to receive a physical quantity distribution inside a member that includes multiple structural elements, and
   a controller configured to identify the structural element to which a voxel included in the member belongs based on the physical quantity distribution, wherein the controller
   generates a plurality of likelihood data indicating likelihood of the structural element to which the voxel belongs based on a plurality of two-dimensional images indicating the physical quantity distribution in a plurality of different cross sections intersecting the voxel,
   calculates a weighted sum of the likelihood based on the plurality of likelihood data generated for each voxel, and
   sets a label for identifying the structural element for which the likelihood is maximized in the voxel based on the weighted sum.

2. The identification device according to claim 1, wherein the controller performs a semantic segmentation on the two-dimensional image to generate the likelihood data.

3. The identification device according to claim 2, wherein the semantic segmentation is performed using a learning model obtained by performing machine learning based on teaching data.

4. The identification device according to any one of claims 1 to 3, wherein the controller generates the plurality of likelihood data based on the plurality of two-dimensional images indicating the physical quantity distribution in three or more cross sections orthogonal to each other.

5. The identification device according to any one of claims 1 to 3, wherein the controller performs threshold processing based on the physical quantity distribution to identify whether or not the voxel is a void.

6. The identification device according to any one of claims 1 to 3, wherein the structural element is at least one of one or more fiber bundles, a matrix, and a void.

7. The identification device according to any one of claims 1 to 3, wherein the receiving unit receives as the physical quantity distribution a distribution representing an absorbance of X-rays at each point inside the member, the distribution being acquired by an X-ray Computed Tomography device.

8. An identification method for identifying a structural element to which a voxel included in a member belongs, the member that includes multiple structural elements, comprising:

   receiving a physical quantity distribution inside the member,
   generating a plurality of likelihood data indicating likelihood of the structural element to which the voxel belongs based on a plurality of two-dimensional images indicating the physical quantity distribution in a plurality of different cross sections intersecting the voxel,
   calculating a weighted sum of the likelihood based on the plurality of likelihood data generated for each voxel, and
   setting a label for identifying the structural element for which the likelihood is maximized in the voxel based on the weighted sum.

9. A non-transitory computer-readable storage medium storing a program for causing a computer to execute processing for identifying a structural element to which a voxel included in a member belongs, the member that includes multiple structural elements, the processing comprising:

receiving a physical quantity distribution inside the member,

generating a plurality of likelihood data indicating likelihood of the structural element to which the voxel belongs based on a plurality of two-dimensional images indicating the physical quantity distribution in a plurality of different cross sections intersecting the voxel,

calculating a weighted sum of the likelihood based on the plurality of likelihood data generated for each voxel, and

setting a label for identifying the structural element for which the likelihood is maximized in the voxel based on the weighted sum.

# FIG. 1

```
                    10
                     ┌──────────┐
                     │ IMAGING  │
                     │   UNIT   │
                     └──────────┘
                          │                              20
  ┌───────────────────────┼────────────────────────────────┐
  │             21         │                                 │
  │      ┌─────────────────▼──┐      ┌──────────────────┐    │
  │      │  RECEIVING UNIT    │      │    DATABASE       │    │
  │      └────────────────────┘      └──────────────────┘    │
  │                │                        │      23        │
  │                └───────────┬────────────┘                │
  │        ┌───────────────────┼─────────────────────┐       │
  │        │ CONTROLLER        │                      │       │
  │  251   │  ┌────────────────────────────┐          │ 25    │
  │        │  │      CROSS SECTION         │          │       │
  │        │  │    DESIGNATING UNIT        │          │       │
  │        │  └────────────────────────────┘          │       │
  │  253   │  ┌────────────────────────────┐          │       │
  │        │  │     TWO-DIMENSIONAL        │          │       │
  │        │  │   IMAGE ACQUIRING UNIT     │          │       │
  │        │  └────────────────────────────┘          │       │
  │  255   │  ┌────────────────────────────┐          │       │
  │        │  │    LIKELIHOOD DATA         │          │       │
  │        │  │    GENERATING UNIT         │          │       │
  │        │  └────────────────────────────┘          │       │
  │  257   │  ┌────────────────────────────┐          │       │
  │        │  │     CALCULATING UNIT       │          │       │
  │        │  └────────────────────────────┘          │       │
  │  259   │  ┌────────────────────────────┐          │       │
  │        │  │    LABEL SETTING UNIT      │          │       │
  │        │  └────────────────────────────┘          │       │
  │        └───────────────────┬─────────────────────┘       │
  │              ┌─────────────┴─────────────┐               │
  │   ┌──────────────────┐          ┌──────────────────┐     │
  │   │   OPERATING      │          │   PRESENTING     │     │
  │   │     UNIT         │          │     UNIT         │     │
  │   └──────────────────┘          └──────────────────┘     │
  │            27                            29              │
  └─────────────────────────────────────────────────────────┘
```

FIG. 2

```
                    ┌─────────────┐
                    │    START    │
                    └──────┬──────┘
                           ↓
              ┌───────────────────────────┐
              │ RECEIVE PHYSICAL QUANTITY  │～S101
              │       DISTRIBUTION         │
              └───────────────┬───────────┘
                              ↓
         ┌──→ ┌───────────────────────────┐
         │    │ DESIGNATE ONE OF PLURALITY │～S103
         │    │     OF CROSS SECTIONS      │
         │    └───────────────┬───────────┘
         │                    ↓
         │    ┌───────────────────────────┐
         │    │         ACQUIRE            │～S105
         │    │   TWO-DIMENSIONAL IMAGE    │
         │    └───────────────┬───────────┘
         │                    ↓
         │    ┌───────────────────────────┐
         │    │  GENERATE LIKELIHOOD DATA  │～S107
         │    └───────────────┬───────────┘
         │                    ↓         S109
         │            ◇───────────────◇
         │           ╱    IS THERE      ╲
         └─────YES───  UNDESIGNATED CROSS
                      ╲    SECTION ?    ╱
                       ◇───────┬───────◇
                           NO  ↓
              ┌───────────────────────────┐
              │ CALCULATE WEIGHTED SUM OF  │～S111
              │        LIKELIHOODS         │
              └───────────────┬───────────┘
                              ↓
              ┌───────────────────────────┐
              │         SET LABEL          │～S113
              └───────────────┬───────────┘
                              ↓
                    ┌─────────────┐
                    │     END     │
                    └─────────────┘
```

# INTERNATIONAL SEARCH REPORT

| International application No. |
|---|
| **PCT/JP2023/037699** |

**A. CLASSIFICATION OF SUBJECT MATTER**

*G06T 7/00*(2017.01)i; *G06V 10/82*(2022.01)i; *G01N 23/046*(2018.01)i; *G01N 23/083*(2018.01)i
FI:   G01N23/046; G01N23/083; G06T7/00 612; G06T7/00 350B; G06V10/82

According to International Patent Classification (IPC) or to both national classification and IPC

**B. FIELDS SEARCHED**

Minimum documentation searched (classification system followed by classification symbols)

G01B,G01C,G01D,G01F,G01G,G01H,G01J,G01K,G01L,G01M,G01N,G01P,G01Q,G01R,G01S,G01T,G01V,G01W,A61B6/00-A61B6/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2016/052489 A1 (IHI CORPORATION) 07 April 2016 (2016-04-07) | 1-9 |
| A | CN 114419284 A (NANCHANG AVIATION UNIV) 29 April 2022 (2022-04-29) | 1-9 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 November 2023** | **21 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/037699**

| Patent document cited in search report | | | Publication date (day/month/year) | Patent family member(s) | | | Publication date (day/month/year) |
|---|---|---|---|---|---|---|---|
| WO | 2016/052489 | A1 | 07 April 2016 | US | 2017/0213357 | A1 | |
| | | | | EP | 3203218 | A1 | |
| CN | 114419284 | A | 29 April 2022 | (Family: none) | | | |

Form PCT/ISA/210 (patent family annex) (January 2015)

**EP 4 618 002 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016052489 A
- JP 2022179731 A